# EUROPEAN PATENT APPLICATION

(11) **EP 3 357 478 A1**
(43) Date of publication of application: **08.08.2018**
(21) Application number: 16850396.9
(22) Date of filing: 30.09.2016
(51) Int. Cl.: A61J 3/06, A61K 8/73, A61K 8/67, A61K 8/02, A61K 9/19, A61Q 19/00, A23L 2/395, A23L 33/00, A23G 3/50, A23G 3/48

(54) **METHOD USING ROLLING MOLD TO PREPARE FREEZE-DRIED EXCIPIENT, AND PRODUCT THEREOF**

(30) Priority: 30.09.2015 CN 201510641264
(71) Applicant: Changzhou Youdo Industrial Investment Co., Ltd., Tianning District, Changzhou Jiangsu 213017 (CN)
(72) Inventor: DONG, Ling, Shunyi District Beijing 101312 (CN)
(74) Representative: Tergau & Walkenhorst Patentanwälte PartGmbB
(86) International application number: PCT/CN2016/101154
(87) International publication number: WO 2017/054772

(57) **Abstract**

The invention relates to the fields of medicines, chemistry, and food products, and specifically relates to a rolling mold combination device, a method using a rolling mold to prepare a freeze-dried excipient, and a product thereof. The method of preparing a freeze-dried excipient has the advantage of being able to engrave any character or pattern at any part of a mold, and correspondingly, can produce the character or pattern at any part of a manufactured product, including a product name or trademark, and such an effect cannot be obtained in existing freeze-dried excipient products. The manufactured product can be designed to be any shape and does not readily break inside packaging, reducing loss of medicines and enabling accurate dosages thereof. The method of preparing a freeze-dried excipient adopts mold-release freeze-drying, and a mold does not re-enter a freeze-drying step, improving production efficiency, decreasing production cost, saving energy and protecting the environment. The mold used in the method can perform continuous production, greatly improving production efficiency, the mold has a long service life, further decreasing production costs, saving energy and protecting the environment.

## Description

### CROSS REFERENCE TO RELATED APPLICATIONS

This application claims the priority of Chinese Patent Application No.201510641264.X, titled with "METHOD USING ROLLING MOLD TO PREPARE FREEZE-DRIED EXCIPIENT, AND PRODUCTS THEREOF", filed with the Chinese Patent Office on September 30, 2015, and the disclosure of which is hereby incorporated by reference.

### FIELD

The present disclosure relates to the field of chemistry, and more particularly to a rolling mold combination device, a method of using the rolling mold to prepare a freeze-dried preparation with an arbitrary shape, and product thereof.

### BACKGROUND

Freeze-drying shaping technology is a shaping technology comprising adding skeleton support agent and binder to a flowable liquid, semi-solid or solid active ingredient, alternatively, the flowable liquid, semi-solid or solid active ingredient per se contains skeleton support agent and binder, and then filling the mixture into the molds for shaping by freeze-drying process. The preparation obtained by freeze-drying shaping technology is called freeze-dried preparation.

This type of preparation is prepared by freeze-drying process, which can protect the thermosensitive ingredient from being damaged and have a fast disintegration and dissolution rate due to a great amount of micropores and channels produced by water sublimation. It has been widely used in a variety of fields such as oral disintegrating tablet, immediate release tablet, chewable tablet, and special cosmetic.

The freeze-dried preparations on the market are mostly single form, and the molds used are traditional molds, that is, ordinary groove-type mold. Such traditional freeze-dried preparations and preparation methods therefor have the following disadvantages:
(1) The shape of preparation is single due to the fixed shape of the molds, and cannot demold or can only demold from single direction; wherein "not demold" means shaping in a packing material having certain shape.
(2) Spherical, ellipsoid, irregular ball type and other special shapes are rare, because it is difficult to produce freeze-dried preparation of special shape by using traditional preparation methods.
(3) The resulting tablet has an acute angle edge, and plurality of tablets having an acute angle edge after demolding are liable to wear after entering in the same package, resulting in inaccurate dosage of the drug.
(4) It is difficult to form a multi-layer structure due to the one-way filling, thus the structure of preparation is single.

### SUMMARY

In view of the above, the present disclosure provides a method of using a rolling mold to prepare a freeze-drying shaped preparation and products thereof. The present disclosure provides a method of preparing a freeze-drying shaped preparation with an arbitrary shape and products thereof. Particularly, the present disclosure relates to a method of preparing a freeze-drying shaped preparation with an arbitrary shape containing mainly active ingredient and binder by using a rolling mold, and products obtained by the method. The present disclosure solves the problem that the shape of the freeze-dried preparation is single. The molds can be designed according to requirements, so that the freeze-dried preparation can be prepared into various shapes as needed, giving the freeze-dried preparation a variety of shapes and structures (double-layer, multi-layer), achieving continuous production, improving production efficiency and reducing costs effectively.

To achieve the above object of the present disclosure, the present disclosure provides the following technical solution:

The present disclosure provides a method of using a rolling mold to prepare a freeze-dried shaped preparation having an arbitrary shape, comprising the following steps:
A. using a relatively rotary rolling mold combination, which comprises a first rolling mold (1), a second rolling mold (2), a filling nozzle (3) and a tray (4); wherein there are a first concave mold (11) with a certain shape and a second concave mold (12) with a certain shape on surfaces of the first rolling mold (1) and the second rolling mold (2); when rolling, the first rolling mold (1) and the second rolling mold (2) rotate relatively to each other, on the surface thereof the first concave mold (11) and the second concave mold (12) when in opposite position are highly matched and close to form a complete shape with a gap of not more than 5mm;
B. precooling the first rolling mold (1) and the second rolling mold (2) at a precooling temperature of not more than 0°C;
C. preparing a solution containing water and a binder into a solution, emulsion or suspension to obtain a primary liquid (5) for the freeze-drying shaped preparation;
D. rotating the first rolling mold (1) and the second rolling mold (2) to fill the primary liquid (5) for the freeze-drying shaped preparation into the concave mold (11) and the concave mold (12) when the lower bottoms of the concave mold (11) and the concave mold (12) on the surface of the first rolling mold (1) and the second rolling mold (2) connect to each other, freeze-drying shaped preparation wherein the filing amount is equal to or close to the total volume of the concave mold (11) and the concave mold (12);
E. freezing and solidifying the freeze-drying shaped preparation primary liquid (5) for the freeze-drying shaped preparation in the precooled first rolling mold (1) and second rolling mold (2);
F. continuing rotating the first rolling mold (1) and the second rolling mold (2); when the concave mold (11) and the concave mold (12) open during the rotation, the frozen and solidified primary liquid (51) for the freeze-drying shaped freeze-drying shaped preparation is released from the concave molds and fall in the tray (4); and
G. performing freeze-drying on the frozen and solidified freeze-drying shaped preparation primary liquid (51) for the freeze-drying shaped preparation to remove solvent and obtain the freeze-drying shaped preparation product.

In step E, if the primary liquid (5) for the freeze-drying shaped preparation is not completely frozen and solidified, a hollow pipe (6) can be further arranged in the first rolling mold (1) and the second rolling mold (2), through which a refrigerant (7) is passed or stored for further cooling the primary liquid until the primary liquid is completely frozen.

The material of the mold described in the preparation method should have certain hardness and a good thermal conductivity. The hardness of the material is represented by indentation hardness, and the indentation hardness thereof is between 0.1N and 100,000N, preferably not more than 90,000N, 80,000N, 70,000N, 60,000N, 50,000N, 40,000N, 30,000N or 20,000N. The thermal conductivity coefficient of the material should be not less than 0.01W/(m.k), where a material having a thermal conductivity coefficient of 0.05W/(m.k) to 1000W/(m.k) is preferred, and a material having a thermal conductivity of 0.2 W/(m.k) to 500W/(m.k) is the most preferred. The material can be one of metal, polymer material, ceramic, glass or the like, or a mixture thereof.

Also, the surface of the mold can be further subjected to surface treatment. The surface treatment of the mold can be coating, electroplating, acid-base washing, polishing, drawing, electrophoresis, PVD and other surface treatment methods, so that the primary liquid for the freeze-drying shaped preparation is well released from the mold after freezing.

The obtained freeze-drying shaped preparation consists essentially of an active ingredient and a binder, in which the weight ratio of the binder to the active ingredient is 1:100 to 100:1.

The weight ratio of the binder to the active ingredient may be further preferably from 1:90 to 90:1, 1:80 to 80:1, 1:70 to 70:1, 1:60 to 60:1, 1:50 to 50:1, 1:40 to 40:1, 1:30 to 30:1, more preferably 1:20 to 20:1,1:10 to 10:1, 1:9 to 9: 1, 1:8 to 8:1, 1:7 to 7:1, and most preferably 1:6 to 6:1, 1:5 to 5:1.

The active ingredient used in the preparation method can be a substance which is water-soluble or water-insoluble, and the active ingredient may be one selected from a chemical pharmaceutical ingredient, an ingredient from traditional Chinese medicine, a natural extract, a bioactive ingredient, and a skin care beneficial ingredient, or a combination thereof.

The active ingredient is not particularly limited and may be selected from but not limited to a composition of one or more of the following ingredients.

### Chemical Pharmaceuticals (Pharmaceutical Active Ingredients):

Antipyretic, analgesic and anti-inflammatory drugs, such as aspirin, diflunisal, salsalate, acetaminophen, indomethacin, ibuprofen, naproxen, ketoprofen, pirprofen, suprofen, flurbiprofen, piroxicam, meloxicam, nimesulide, benzbromarone, etc.;
central stimulants, such as pemoline, adrafinil, piracetam, etc.;
drug for treating migraine, such as sumatriptan succinate;
analgesics, such as rotundine, buprenorphine, pentazocine, naloxone, etc.;
drugs for treating Parkinson's disease and senile dementia, such as levodopa, compound carbidopa, compound benserazide, amantadine hydrochloride, piribedil, profenamine, donepezil, huperzine-A, etc.;
antipsychataxia drugs, such as chlorpromazine, promethazine, pethidine, thioridazine, chlorprothixene, clozapine, sulpiride, tiapride, penfluridol, risperidone, etc.;
antiepileptic and anticonvulsant drugs, such as phenytoin sodium, carbamazepine, primidone, gabapentin, lamotrigine, sodium valproate, clonazepam, etc.;
sedative hypnotics, such as diazepam, nitrazepam, oxazepam, lorazepam, phenobarbital, etc.;
cholinesterase inhibitor, such as scopolamine, etc.;
antiarrhythmics, such as propiopyridine, tocainide, mexiletine, ethmozine, phenytoin sodium, propafenone, amiodarone, etc.;
anti-angina pectoris and anti-atherosclerosis drugs, such as propranolol, nifedipine, gemfibrozil, bezafibrate, lovastatin, simvastatin, pravastatin, etc.;
antihypertensive drugs, such as enalapril, captopril, hydrochlorothiazide, amlodipine, etc.;
adrenergic receptor blockers, such as acebutolol, alprenolol, etc.;
corticosteroids, such as betamethasone, cortisone acetate, etc.;
antidiabetic drugs, such as repaglinide, etc.;
anti-thyroid drugs, such as propylthiouracil, carbimazole, methimazole, etc.;
antihistamines, such as cetirizine hydrochloride, loratadine, etc.;
autacoids, such as dinoprostone, alprostadil, betahistine, etc.;
digestive system drugs, such as butylbromide scopolamine, granisetron hydrochloride, etc .;
blood system drugs, such as EPO, cobamamide, etc.;
urinary system drugs, such as azosemide, furosemide, etc.;
reproductive system drugs, such as estrogen, nandrolone phenylpropionate, etc.;
antiparasitic drugs, such as albendazole, cambendazole, etc.;
antineoplastic drugs, such as aminoglutethimide, amsacrine, etc.;
antimicrobial drugs, such as ampicillin, sulbenicillin sodium, etc.;
antibiotics such as amoxicillin, cephalexin, cefprozil, cefuroxime axetil, roxithromycin, erythromycin ethylsuccinate, josamycin and so on.

### Ingredients from Traditional Chinese Medicine:

active ingredient monomer from traditional Chinese medicine, such as breviscapine, artemisinin, huperzine-A, corydalis B, etc.;
single Chinese medicine extract and compound Chinese medicine extract, such as tanshinone extract, total salvianolicacid extract, compound Danshen dropping pill extract, compound Niuhuangshangqing pill extract, total saponin from ginseng stem and leaf, *Rhizoma Menispermi* extract, total saponin of ginseng, total saponin of American ginseng, breviscapine, *Sarcandrae Herba* extractum, total notoginsenoside, *Artemisia capillaris* extract, *Rhei Radix et Rhizoma* extractum, andrographolide, hawthorn leaf extract, total asiaticoside, ginkgo leaf extract, and so on.

### Natural Plant Extracts:

aloe extract, Chinese yam extract, cowberry extract, bitter gourd extract, echinacea extract, feverfew extract, mangosteen extract, pine needle and pine bark extract, acai berry extract, mulberry extract, elderberry extract, cranberry extract, astaxanthin, lycopene, green tea extract, grape seed and grape skin extract, glabridin, paeoniflorin, glycyrrhizicflavone, tree peony bark extract, and so on.

### Bioactive Ingredients:

EGF, bFGF, aFGF, KGF, IGF, NGF, TGF, HGH and the like.

### Skin Care Beneficial Ingredients:

vitamin A, vitamin B1, vitamin B2, vitamin B3, vitamin B6, vitamin B12, vitamin C, vitamin D, vitamin E, vitamin K, coenzyme, protease, metallothionein, pearl and its hydrolysate, milk and its extract, pollen and its extract, royal jelly, propolis, and so on.

The binder is an edible or pharmaceutically acceptable water-soluble polymeric material, which may be polysaccharide, polypeptide, protein, or synthetic polymer, or modified natural polymeric material or a mixture thereof. The binders include, but are not limited to, gelatin (gelatin, fish gelatin, bird gelatin, hydrolyzed gelatin, etc.), cellulose ether (methylcellulose, carboxymethylcellulose, hydroxypropylmethylcellulose, carboxyethylmethylcellulose, etc.), modified starch (pullulan, hydroxymethyl starch, etc.), hyaluronic acid, albumin, dextran, chitosan and its different molecular weight products, sodium alginate, PVP, PVA, polyethylene glycol, arabic gum, guar gum, xanthan gum, konjac gum, carrageenan, carbomer, agar, carrageenin, pectin or a combination thereof. The gum binder is collagen, gelatin, hydrolyzed gelatin, arabic gum, xanthan gum, carrageenan, pectin, konjac gum, carrageenin, locust bean gum, gum, locust bean gum; the cellulose ether binder is carboxymethylcellulose, carboxyethylcellulose, hydroxyethylmethylcellulose, hydroxypropylmethylcellulose or the like; the modified starch-derived binder is selected from pullulan, hydroxypropyl starch, hydroxypropylmethyl starch, pregelatinized starch, amylose, carboxymethyl starch, hydroxyethyl starch, hydroxypropyl starch, etc.; the poly amino acid is selected from polyglutamic acid, polyalanine, polylysine and the like; and the polysaccharide is selected from fucoidan, inulin and the like.

The freeze-drying shaped preparation may further comprise other adjuvant such as a backbone support agent, an antioxidant, a flavoring agent and essence, a transdermal or penetration adsorption enhancer, a pH adjusting agent, and the like. The content of other adjuvant may be 0.1% to 5% of the obtained freeze-drying shaped preparation, preferably 0.1% to 3%.

The backbone support agent includes but not limited to sugar (such as maltose, trehalose, etc.), sugar alcohols (such as mannitol, sorbitol), amino acid having 2-12 carbon atoms (such as glycine, alanine, glutamic acid, etc.), as well as inorganic salt (such as sodium phosphate, aluminum silicate, etc.) and other substances.

The antioxidant includes but not limited to one of vitamin C and its derivatives, anthocyanin, resveratrol, and plant-derived polyphenol, or a mixture thereof.

The flavoring agent and essence includes but not limited to one of a essence with mint flavor, chocolate flavor, fruity flavor, vanilla flavor, coffee flavor, tea flavor, corn flavor, lemon flavor and milk flavor, or a mixture thereof.

The transdermal or penetration absorption enhancer includes but not limited to one of lecithin, saponin, sodium lauryl alcohol acid, azone, tween and span, or a mixture thereof.

The pH adjusting agent includes but not limited to one of citric acid, tartaric acid, sodium bicarbonate, carbonate, sodium carbonate and phosphate, or a mixture thereof.

The product prepared by the method of the present disclosure may be of any shape depending on the cavity enclosed by the mold. Preferably, the shape is tablet shape, capsule shape, soft capsule shape, spherical shape, ellipsoid shape or a shape of various characters, animals, plants, foods, graphic logos or cartoon characters. The product obtained by the method of the present disclosure may have no sharp corner and edge depending on the shape of the mold.

The freeze-drying shaped preparation prepared by the present disclosure has the following advantages: (1) Any part of the mold can be engraved with words or patterns, which may be the product name or trademark. Correspondingly, words or patterns will also appear on any part of the prepared product, which is not available in the existing freeze-drying shaped preparation products. (2) The resulting product can be designed to be of any shapes which are not easy to be broken in the package, reducing drug loss and allowing accurate dosage of the drug. (3) The freeze-drying shaped preparation in the present disclosure is prepared by demolding and freeze-drying process, so the molds do not enter the freeze-drying process, improving production efficiency, reducing production costs and thus being energy saving and environmental protection. (4) The molds used in the method of the present disclosure can be used in continuous production, greatly improving the production efficiency, prolonging the mold life, further reducing the production cost and thus being energy saving and environmental protection.

### BRIEF DESCRIPTION OF DRAWINGS

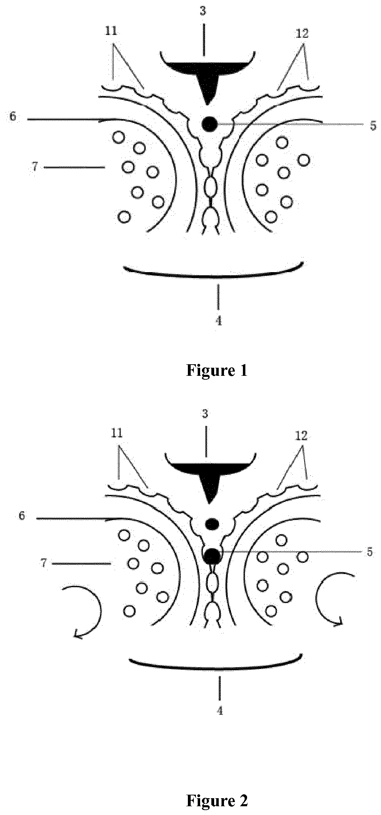

Figure 1 is the sectional view of the components of a rolling mold equipment for production.

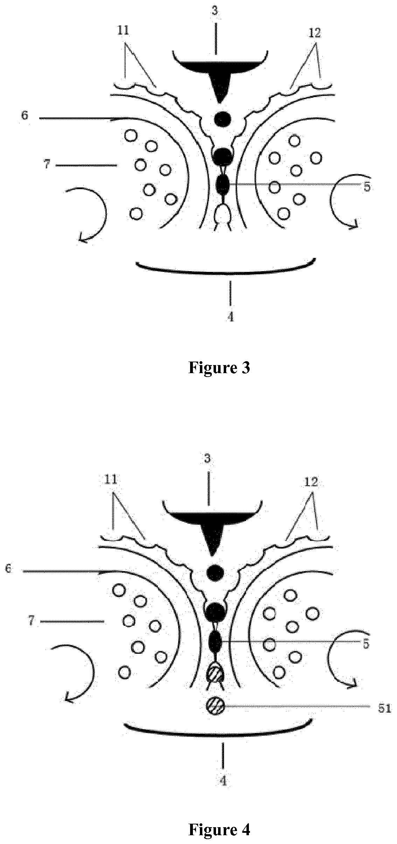

Figures 2-4 are production flow charts of preparing a freeze-drying shaped preparation by using rolling mold method.

### DETAILED DESCRIPTION

The present disclosure discloses a rolling mold combination device, a method of using the rolling mold to prepare a freeze-drying shaped preparation and product thereof. Those skilled in the art can achieve it by modifying the process parameters appropriately in view of the contents of the present disclosure. It should be indicated particularly that all similar substitutions and modifications will be apparent to those skilled in the art and are considered to be in the scope of the present disclosure. The methods and applications of the present disclosure have been described by way of preferred embodiments, and it will be apparent to those skilled in the art that appropriate changes and combinations of the methods and applications described herein may be made without departing from the content, spirit and scope of the present disclosure, so as to realize and apply the technology of the present disclosure.

The materials and reagents used in the rolling mold combination device, the method of using the rolling mold to prepare a freeze-drying shaped preparation with an arbitrary shape and the resulting product of the present disclosure are all commercially available in the market.

The present disclosure is further described with reference to the following examples:

### Example 1

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to cowberry extract and pullulan (cowberry extract : pullulan = 5:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; filling the freeze-drying shaped preparation primary liquid (5) into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12)opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a cowberry instant solid beverage product.

### Example 2

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a capsule shape, respectively; when the concave mold (11) and the concave mold (12) were connected to each other, a complete capsule shape with a gap of 0.5mm was formed;
B. the first rolling mold (1) and the second rolling mold (2) were precooled to a precooling temperature of 0°C;
C. a mixture of GTCC, vitamin D and lecithin (GTCC : vitamin D : lecithin = 5:1:1) was completely stirred and vacuum degassing was performed to obtain composition I; water was added to hydrolyzed gelatin and pullulan (hydrolyzed gelatin : pullulan = 1:1), stirred completely and centrifuge was performed to remove gas to obtain composition II; the mass of the composition I was 0.1% of the total amount of the freeze-drying shaped preparation;
D. the first rolling mold (1) and the second rolling mold (2) were rotated to make the bottoms of the concave mold (11) and the concave mold (12) on the surface connected to each other; the composition I and the composition II were filled sequentially into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a capsule shape left the concave molds and fell in the tray (4); and
G. freeze-drying was performed on the frozen and solidified freeze-drying shaped preparation primary liquid (51) in the tray to remove solvent and obtain an oral nutritional supplements product.

### Example 3

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a certain shape, which can form a complete mango shape, and the shapes of the first rolling mold (1) and the second rolling mold (2) are asymmetric; when the concave mold (11) and the concave mold (12) were connected to each other, a complete mango shape with a gap of 0.3mm was formed;
B. the first rolling mold (1) and the second rolling mold (2) were precooled to a precooling temperature of -10°C;
C. water was added to mango juice concentrate and trehalose (mango juice concentrate: trehalose = 3:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a mango shape left the concave molds and fell in the tray (4); and
G. freeze-drying was performed on the frozen and solidified freeze-drying shaped preparation primary liquid (51) in the tray to remove solvent and obtain a mango-flavored candy product.

### Example 4

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a certain shape, respectively; when the concave mold (11) and the concave mold (12) were connected to each other, a complete water-drop shape with a gap of 0.2mm was formed;
B. refrigerant (7)was filled into the hollow pipe (6) of the first rolling mold (1) and the second rolling mold (2) to precool the rolling mold to a temperature of -30°C;
C. water was added to vitamin C and pullulan (vitamin C : pullulan = 10:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a water-drop shape left the concave molds and fell in the tray (4); and
G. freeze-drying was performed on the frozen and solidified freeze-drying shaped preparation primary liquid (51) in the tray to remove solvent and obtain a vitamin skin care product.

### Example 5

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to dextran and *Tremella Fuciformis* extract (dextran:*Tremella Fuciformis* extract = 1:100) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a moisturizing cosmetic.

### Example 6

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to *Notoginseng Radix Et Rhizoma* extract and pullulan (*Notoginseng Radix Et Rhizoma* extract : pullulan = 100:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51)with a sphere shape left the concave molds and fell in the tray (4); and
G. freeze-drying was performed on the frozen and solidified freeze-drying shaped preparation primary liquid (51) in the tray to remove solvent and obtain a *Notoginseng Radix Et Rhizoma* whitening cosmetic.

### Example 7

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to hyaluronic acid and dextran (hyaluronic acid : dextran = 6:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51)with a sphere shape left the concave molds and fell in the tray (4); and
G. freeze-drying was performed on the frozen and solidified freeze-drying shaped preparation primary liquid (51) in the tray to remove solvent and obtain a moisturizing cosmetic.

### Example 8

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to arbutin and PVPK (arbutin : PVPK = 1:6) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51)with a sphere shape left the concave molds and fell in the tray (4); and
G. freeze-drying was performed on the frozen and solidified freeze-drying shaped preparation primary liquid (51) in the tray to remove solvent and obtain a whitening skin care cosmetic.

### Example 9

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to *Ginseng Radix Et Rhizoma* extract and caprylic/capric triglyceride (*Ginseng Radix Et Rhizoma* extract :caprylic/capric triglyceride = 50:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a Ginseng skin care product.

### Example 10

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to fish collagen and mannitol (fish collagen: mannitol= 1:50) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a skin care product.

### Example 11

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to *Dendrobii Officinalis Caulis* extract and carbomer (*Dendrobii Officinalis Caulis* extract: carbomer= 1:50) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a freeze-drying shaped preparation.

### Example 12

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to human oligopeptide-landpullulan (human oligopeptide-1 : pullulan = 5:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a skin care product.

### Example 13

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to pearl powder and vitamin C (pearl powder: vitamin C = 10:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a whitening skin care cosmetic.

### Example 14

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to lotus extract and trehalose (lotus extract: trehalose = 1:10) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a skin care product.

### Example 15

A. A relatively rotary rolling mold combination was used, which comprises a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to aloe vera extract and polyglutamic acid (aloe vera extract: polyglutamic acid = 90:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a moisturizing skin care product.

### Example 16

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 1mm was formed;
B. liquid nitrogen (7) was filled into the hollow pipe (6) of the first rolling mold (1) and the second rolling mold (2) to precool the rolling mold to a temperature of -20°C;
C. water was added to tremella polysaccharides and xanthan gum (tremella polysaccharides: xanthan gum = 1:90) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a skin care product.

### Example 17

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) of a capsule shape, respectively; when the concave mold (11) and the concave mold (12) were connected to each other, a complete capsule shape with a gap of 0.5mm was formed;
B. the first rolling mold (1) and the second rolling mold (2) were precooled to a precooling temperature of 0°C;
C. a mixture of GTCC, vitamin D and lecithin (GTCC : vitamin D : lecithin = 5:1:1) was completely stirred and vacuum degassing was performed to obtain composition I; water was added to hydrolyzed gelatin and pullulan (hydrolyzed gelatin : pullulan = 1:70) and stirred completely; centrifuge was performed to remove gas to obtain composition II; the mass of the composition I was 5% of the total amount of the freeze-drying shaped preparation;
D. the first rolling mold (1) and the second rolling mold (2) were rotated to make the bottoms of the concave mold (11) and the concave mold (12) on the surface connected to each other; the composition I and the composition II were filled sequentially into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a capsule shape left the concave molds and fell in the tray (4); and
G. freeze-drying was performed on the frozen and solidified freeze-drying shaped preparation primary liquid (51) in the tray to remove solvent and obtain an oral nutritional supplements product.

### Example 18

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) of a capsule shape, respectively; when the concave mold (11) and the concave mold (12) were connected to each other, a complete capsule shape with a gap of 0.5mm was formed;
B. the first rolling mold (1) and the second rolling mold (2) were precooled to a precooling temperature of 0°C;
C. a mixture of GTCC, vitamin D and lecithin (GTCC : vitamin D : lecithin = 5:1:1) was completely stirred and vacuum degassing was performed to obtain composition I; water was added to hydrolyzed gelatin and pullulan (hydrolyzed gelatin : pullulan = 70:1) and stirred completely; centrifuge was performed to remove gas to obtain composition II; the mass of the composition I was 0.1% to 5% of the total amount of the freeze-drying shaped preparation, preferably 0.1% to 3%;
D. the first rolling mold (1) and the second rolling mold (2) were rotated to make the bottoms of the concave mold (11) and the concave mold (12) on the surface connected to each other; the composition I and the composition II were filled sequentially into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a capsule shape left the concave molds and fell in the tray (4); and
G. freeze-drying was performed on the frozen and solidified freeze-drying shaped preparation primary liquid (51) in the tray to remove solvent and obtain an oral nutritional supplements product.

### Example 19

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) of a capsule shape, respectively; when the concave mold (11) and the concave mold (12) were connected to each other, a complete capsule shape with a gap of 0.5mm was formed;
B. the first rolling mold (1) and the second rolling mold (2) were precooled to a precooling temperature of 0°C;
C. a mixture of GTCC, vitamin D and lecithin (GTCC : vitamin D : lecithin = 5:1:1) was completely stirred and vacuum degassing was performed to obtain composition I; water was added to hydrolyzed gelatin and pullulan (hydrolyzed gelatin : pullulan = 40:1) and stirred completely; centrifuge was performed to remove gas to obtain composition II; the mass of the composition I was 3% of the total amount of the freeze-drying shaped preparation;
D. the first rolling mold (1) and the second rolling mold (2) were rotated to make the bottoms of the concave mold (11) and the concave mold (12) on the surface connected to each other; the composition I and the composition II were filled sequentially into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a capsule shape left the concave molds and fell in the tray (4); and
G. freeze-drying was performed on the frozen and solidified freeze-drying shaped preparation primary liquid (51) in the tray to remove solvent and obtain an oral nutritional supplements product.

### Example 20

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) of a capsule shape, respectively; when the concave mold (11) and the concave mold (12) were connected to each other, a complete capsule shape with a gap of 0.5mm was formed;
B. the first rolling mold (1) and the second rolling mold (2) were precooled to a precooling temperature of 0°C;
C. a mixture of GTCC, vitamin D and lecithin (GTCC : vitamin D : lecithin = 5:1:1) was completely stirred and vacuum degassing was performed to obtain composition I; water was added to hydrolyzed gelatin and pullulan (hydrolyzed gelatin : pullulan = 1:40) and stirred completely; centrifuge was performed to remove gas to obtain composition II; the mass of the composition I was 1% of the total amount of the freeze-drying shaped preparation;
D. the first rolling mold (1) and the second rolling mold (2)were rotated to make the bottoms of the concave mold (11) and the concave mold (12) on the surface connected to each other; the composition I and the composition II were filled sequentially into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a capsule shape left the concave molds and fell in the tray (4); and
G. freeze-drying was performed on the frozen and solidified freeze-drying shaped preparation primary liquid (51) in the tray to remove solvent and obtain an oral nutritional supplements product.

### Example 21

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 0.8mm was formed;
B. liquid nitrogen (7) was filled into the hollow pipe (6) of the first rolling mold (1) and the second rolling mold (2) to precool the rolling mold to a temperature of -30°C;
C. water was added to brufen and xanthan gum (brufen: xanthan gum= 1:1) to prepare a solution; centrifuge was performed to remove gas to obtain a freeze-drying shaped preparation primary liquid;
D. the first rolling mold (1) and the second rolling mold (2)were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5)was frozen and solidified in the precooled first rolling mold (1) and second rolling mold (2);
F. the first rolling mold (1) and the second rolling mold (2)were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a brufen medicine.

### Example 22

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) of a hemispherical shape, respectively; when the concave mold (11) and the concave mold (12) were connected to each other, a complete sphere shape with a gap of 0.8mm was formed;
B. liquid nitrogen (7) was filled into the hollow pipe (6) of the first rolling mold (1) and the second rolling mold (2) to precool the rolling mold to a temperature of -18°C;
C. water was added to acetaminophen and pullulan (acetaminophen: pullulan= 5:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a sphere shape left the concave molds and fell in the tray (4); and
G. freeze-drying was performed on the frozen and solidified freeze-drying shaped preparation primary liquid (51) in the tray to remove solvent and obtain a skin care product.

### Example 23

A. A relatively rotary rolling mold combination was used, which comprised a first rolling mold (1), a second rolling mold (2), a filling nozzle (3) and a tray (4); wherein surfaces of the first rolling mold (1) and the second rolling mold (2) have concave mold (11) and concave mold (12) with a certain shape; when the concave mold (11) and the concave mold (12) were connected to each other, a complete water-drop shape with a gap of 0.2mm was formed;
B. liquid nitrogen (7) was filled into the hollow pipe (6) of the first rolling mold (1) and the second rolling mold (2) to precool the rolling mold to a temperature of -30°C;
C. water was added to ascorbic acid and pullulan (ascorbic acid: pullulan= 10:1) to prepare a solution; centrifuge was performed to remove gas to obtain a freeze-drying shaped preparation primary liquid;
D. the first rolling mold (1) and the second rolling mold (2) were rotated to make the bottoms of the concave mold (11) and the concave mold (12) on the surface connected to each other; the freeze-drying shaped preparation primary liquid (5) was filled into the concave mold (11) and the concave mold (12) at an amount equal to or close to the total volume of the concave molds (11) and (12);
E. the freeze-drying shaped preparation primary liquid (5) was frozen and solidified in the precooled first rolling mold (1) and second rolling mold (2);
F. the first rolling mold (1) and the second rolling mold (2) were continued to rotate; when the concave molds (11) and (12) opened during the rotation, the frozen and solidified freeze-drying shaped preparation primary liquid (51) with a water-drop shape left the concave molds and fell in the tray (4); and
G. freeze-drying was performed on the frozen and solidified freeze-drying shaped preparation primary liquid (51) in the tray to remove solvent and obtain a vitamin skin care product.

### Example 24

Based on maximum output of 8h, the maximum output of the production method in the prior art is 300,000 tablets/day; while the maximum output of the method provided by the present disclosure is 1,600,000 tablets/day.

Based on the above daily output, the comparison of production cost between the prior art production method and the method provided by the present disclosure is shown in Table 1.

**Table 1. Results of productions**

| Prior Art Production Method | | Method Provided by the Present Disclosure | |
|---|---|---|---|
| Feasibility | Production Cost | Feasibility | Production Cost |
| No | 0.2-0.3yuan/tablet | Yes | 0.05-0.1 yuan/tablet |

A rolling mold combination device, a method of using the rolling mold to prepare a freeze-drying shaped preparation and product thereof provided by the present disclosure are described in detail in the above. The principles and embodiments of the present disclosure have been described with reference to specific examples, and the description of the above embodiments is merely for the purpose of understanding the method of the invention and its core idea. It should be noted that it will be apparent to one of ordinary skill in the art that various improvements and modifications may be made to the present disclosure without departing from the principles of the invention, which fall within the scope of the claims of the present disclosure.

## Claims

1. A relatively rotary rolling mold combination device, comprising: a first rolling mold (1), a second rolling mold (2), a filling nozzle (3) and a tray (4); wherein there are a first concave mold (11) with a certain shape and a second concave mold (12) with a certain shape on surfaces of the first rolling mold (1) and the second rolling mold (2); when rolling, the first rolling mold (1) and the second rolling mold (2) rotate relatively to each other, on the surface thereof the first concave mold (11) and the second concave mold (12) when in opposite position are highly matched and close to form a complete shape with a gap of not more than 5mm.

2. A method of manufacturing a freeze-drying shaped preparation having an arbitrary shape, comprising using the rolling mold combination device according to claim 1.

3. A method of manufacturing a freeze-drying shaped preparation having an arbitrary shape by using the relatively rotary rolling mold combination device according to claim 1, comprising the following steps:
A. using a relatively rotary rolling mold combination, which comprises a first rolling mold (1), a second rolling mold (2), a filling nozzle (3) and a tray (4); wherein there are a first concave mold (11) with a certain shape and a second concave mold (12)with a certain shape on surfaces of the first rolling mold (1) and the second rolling mold (2); when rolling, the first rolling mold (1) and the second rolling mold (2) rotate relatively to each other, on the surface thereof the first concave mold (11) and the second concave mold (12) when in opposite position are highly matched and close to form a complete shape with a gap of not more than 5mm;
B. precooling the first rolling mold (1) and the second rolling mold (2) at a precooling temperature of not more than 0°C;
C. preparing a solution containing water and a binder into a solution, emulsion or suspension to obtain a primary liquid (5) for the freeze-drying shaped preparation;
D. rotating the first rolling mold (1) and the second rolling mold (2) to fill the primary liquid (5) for the freeze-drying shaped preparation into the concave mold (11) and the concave mold (12) when the lower bottoms of the concave mold (11) and the concave mold (12) on the surface of the first rolling mold (1) and the second rolling mold (2) connect to each other, freeze-drying shaped preparation wherein the filing amount is equal to or close to the total volume of the concave mold (11) and the concave mold (12);
E. freezing and solidifying the freeze-drying shaped preparation primary liquid (5) for the freeze-drying shaped preparation in the precooled first rolling mold (1) and second rolling mold (2);
F. continuing rotating the first rolling mold (1) and the second rolling mold (2); when the concave mold (11) and the concave mold (12) open during the rotation, the frozen and solidified primary liquid (51) for the freeze-drying shaped freeze-drying shaped preparation is released from the concave molds and fall in the tray (4); and
G. performing freeze-drying on the frozen and solidified freeze-drying shaped preparation primary liquid (51) for the freeze-drying shaped preparation to remove solvent and obtain the freeze-drying shaped preparation product.

4. The method according to claim 3, wherein the solution containing water and binder in step C further comprises an active ingredient, including but not limited to a chemical pharmaceutical ingredient, an ingredient from traditional Chinese medicine, a natural extract, a bioactive ingredient, an oral food supplement and a skin care beneficial ingredient, which is beneficial to human and animal, or a combination thereof; wherein the weight ratio of the binder to the active ingredient is 1:100 to 100:1, preferably the weight ratio of the binder to the active ingredient is 1:90 to 90:1, 1:80 to 80:1, 1:70 to 70:1, 1:60 to 60:1, 1:50 to 50:1, 1:40 to 40:1, 1:30 to 30:1, more preferably 1:20 to 20:1,1:10 to 10:1, 1:9 to 9: 1, 1:8 to 8:1, 1:7 to 7:1, and most preferably 1:6 to 6:1, 1:5 to 5:1.

5. The method according to claim 3 or 4, wherein the solution containing water and binder in step C further comprises other adjuvant selected from a backbone support agent, an antioxidant, a flavoring agent and essence, a transdermal or penetration adsorption enhancer and pH adjusting agent, or a mixture thereof.

6. The method according to claim 5, wherein the backbone support agent includes but not limited to sugar, sugar alcohol, amino acid having 2-12 carbon atoms and inorganic salt; the antioxidant is selected from one of vitamin C and its derivative, anthocyanin, resveratrol and plant-derived polyphenol, or a mixture thereof; the flavoring agent and essence is selected from an essence with mint flavor, chocolate flavor, fruity flavor, vanilla flavor, coffee flavor, tea flavor, corn flavor, lemon flavor and milk flavor, or a mixture thereof; the transdermal or penetration absorption enhancer is selected from one of lecithin, saponin, sodium lauryl alcohol acid, azone, tween and span, or a mixture thereof; the pH adjusting agent is selected from one of citric acid, tartaric acid, sodium bicarbonate, carbonate, sodium carbonate and phosphate, or a mixture thereof; wherein
the sugar is selected from one of maltose and trehalose, or a mixture thereof;
the sugar alcohol is selected from one of mannitol and sorbitol, or a mixture thereof;
the amino acid having 2-12 carbon atoms is selected from one of glycine, alanine and glutamic acid, or a mixture thereof;
the inorganic salt is selected from one of sodium chloride, sodium phosphate and aluminum silicate, or a mixture thereof;
the content of the other adjuvant is 0.1% to 5% of the sphere shaped freeze-drying shaped preparation, preferably 0.1% to 3%.

7. The method according to any one of claims 3 to 6, wherein in step E, the precooled molds can freeze and solidify the primary liquid for the freeze-drying shaped preparation; if the primary liquid freeze-drying shaped preparation is not completely frozen and solidified, a hollow pipe (6) may be further arranged in the first rolling mold (1) and the second rolling mold (2), through which a refrigerant (7) is passed or stored for further cooling the primary liquid until the primary liquid is completely frozen.

8. The method according to any one of claims 3 to 7, wherein the mold is made of a material having an indentation hardness of equal to and above 0.1N and a thermal conductivity coefficient of equal to and above 0.01W/(m.k), wherein the material is selected from one of metal, polymer material, ceramic and glass, or a mixture thereof.

9. The method according to any one of claims 3 to 8, wherein the surface of the mold may be further subjected to surface treatment so that the primary liquid for the freeze-drying shaped preparation can be well released from the mold after freezing.

10. The method according to any one of claims 3 to 9, wherein the binder is an edible or pharmaceutically acceptable water-soluble polymeric material, or a mixture thereof, including polysaccharide, polypeptide, protein, synthetic polymer, modified natural polymeric material or a mixture thereof, or natural material containing water-soluble polymer, or a mixture thereof.

11. The method according to any one of claims 3 to 10, wherein the binder includes but not limited to gelatin, cellulose ether, modified starch, hyaluronic acid, albumin, dextran, chitosan and product thereof with different molecular weights, sodium alginate, PVP, PVA, polyethylene glycol, arabic gum, guar gum, xanthan gum, konjac gum, carrageenan, carbomer, agar, carrageenan and pectin, or a combination thereof, and natural material containing water-soluble polymer; wherein
the gelatin includes one of gelatin, fish gelatin, bird gelatin and hydrolyzed gelatin, or a mixture thereof;
the cellulose ether includes one of methylcellulose, carboxymethyl cellulose, hydroxypropylmethyl cellulose and carboxyethylmethyl cellulose, or a mixture thereof;
the modified starch includes one of pullulan and hydroxymethyl starch, or a mixture thereof.

12. A product, prepared by the method according to any one of claims 3 to 11.

13. The product according to claim 12, which has an arbitrary shape.

14. The product according to claim 13, which has no sharp edge and corner in shape.

15. The product according to claim 13 or 14, which is in a shape of tablet shape, capsule shape, soft capsule shape, sphere shape, ellipsoid shape or a shape of various characters, animals, plants, foods, graphic logos or cartoon characters.
